Europäisches Patentamt

**European Patent Office**

Office européen des brevets

(19)

(11) Veröffentlichungsnummer: **0 227 921**
**B1**

(12) **EUROPÄISCHE PATENTSCHRIFT**

(45) Veröffentlichungstag der Patentschrift:
21.02.90

(21) Anmeldenummer: 86115350.0

(22) Anmeldetag: 05.11.86

(51) Int. Cl.⁵: **G 01 N 33/557,** G 01 N 33/563,
G 01 N 33/577, G 01 N 33/537,
G 01 N 33/543, G 01 N 33/574

(54) Verfahren zur Bestimmung einer immunologisch bindefähigen Substanz.

(30) Priorität: 05.11.85 DE 3539215

(43) Veröffentlichungstag der Anmeldung:
08.07.87 Patentblatt 87/28

(45) Bekanntmachung des Hinweises auf die Patenterteilung:
21.02.90 Patentblatt 90/8

(84) Benannte Vertragsstaaten:
AT BE CH DE ES FR GB GR IT LI LU NL SE

(56) Entgegenhaltungen:
DE-A- 3 205 849
DE-A- 3 425 008
US-A- 4 329 213

(73) Patentinhaber: BOEHRINGER MANNHEIM GMBH,
Patentabteilung, Abt. E Sandhofer
Strasse 112-132 Postfach 31 01 20,
D-6800 Mannheim 31 Waldhof (DE)

(72) Erfinder: Kaspar, Klaus Peter, Dr. rer. nat., Calle
Fulgencio R. Moreno 5309, Asuncion (PY)

(74) Vertreter: Weickmann, Heinrich, Dipl.-Ing. et al,
Patentanwälte Dipl.-Ing. H.Weickmann Dipl.-Phys.Dr.
K.Fincke Dipl.-Ing. F.A.Weickmann Dipl.-Chem. B. Huber
Dr.-Ing. H. Liska Dipl.-Phys.Dr. J. Prechtel
Postfach 860820, D-8000 München 86 (DE)

ACTORUM AG

**Beschreibung**

Die Erfindung betrifft ein Verfahren und ein Reagenz zur Bestimmung eines bindefähigen Analyten nach dem Prinzip des zweiseitigen heterogenen Immunoassays.

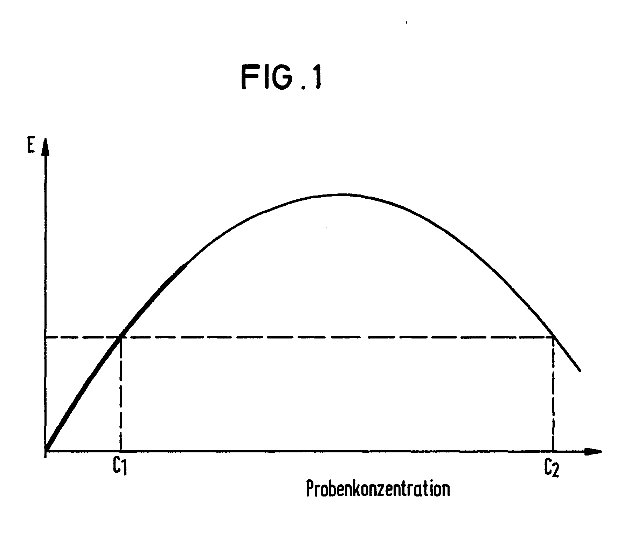

Zweiseitige Immunoassays (Sandwich- Test) zeigen im Vergleich zu den kompetitiven Immunoassays bezüglich Genauigkeit, Empfindlichkeit und schneller Durchführbarkeit deutliche Vorteile. Bei der Variation von Konzentrationen eines Reagenzes, Einsatzstoffes oder einer Probe durchläuft bei immunochemischen Meßverfahren der Meßwert ein Maximum. Dieser Effekt wird als Hook-Effekt bezeichnet. Bei Sandwich-Immunoassays mit Festphasenbeteiligung wird zuerst bei steigender Analytmenge ein ebenfalls steigendes Meßsignal beobachtet, das bei weiterer Zunahme des Analyten dann wieder abnehmen kann. In diesem Fall kann ein bestimmtes Meßergebnis durch zwei verschiedene Analytmengen hervorgerufen werden (siehe Fig. 1). Dieser von der Analytmenge abhängige Effekt wird als «High Dose» Hook-Effekt bezeichnet und schränkt die Anwendung von Sandwich-Assays trotz der oben erwähnten Vorteile ein.

Für den Hook-Effekt werden verschiedene Gründe, wie Heterogenität der in den Rezeptoren vorhandenen Antikörper bzw. unvollständiges Waschen, angegeben (siehe D. Rodbard et al, Immunochemistry 1978, Vol. 15, S. 77-82). Unabhängig davon muß jedoch aus theoretischen Überlegungen der Hock-Effekt zwangsläufig bei allen Einschritt-Sandwich-Tests auftreten. Als Einschritt-Test werden hier alle Tests bezeichnet, bei denen der Analyt mit 2 für ihn spezifischen, mit dem Analyten ein «Sandwich» bildenden Rezeptoren, in derselben Lösung zur Reaktion gebracht und dabei oder anschließend an ein unlösliches Trägermaterial fixiert wird, im Gegensatz zur sequentiellen Reaktionsführung, bei denen nach Reaktion des Analyten mit einem ersten spezifischen Rezeptor und Fixierung des gebildeten Komplexes an eine feste Phase nicht gebundener Analyt durch Waschen der festen Phase vor der Reaktion mit einem zweiten spezifischen markierten Rezeptor entfernt wird. In den Einschrittsandwich-Tests tritt zwangsläufig dann der Hook-Effekt auf, wenn beide spezifischen Rezeptoren im Unterschuß zum Analyten sind, so daß nur ein Teil des Analyten mit Konjugat komplexiert ist und von diesen Komplexen auch nur ein Teil auf der Festphase fixiert wird.

Auch alle Proteinbestimmungen mit Hilfe des DASP-Sandwich-Prinzips, bei welchem die beiden spezifischen Rezeptoren löslich sind und ihr Komplex mit dem Analyten über einen weiteren Rezeptor an der festen Phase fixiert wird sind im obigen Sinne Einschritt-Tests und zeigen einen Hook-Effekt. Dies kann unter anderem bei Tumormarkern, wie z.B. AFP, CEA, hCG, IgE etc. bei bereits hochpathologischen Proben Ergebnisse im Normalbereich vortäuschen. Daher wird häufig für medizinische Bestimmungen die Verwendung des Einschritt-Sandwich-Verfahrens grundsätzlich abgelehnt. Ein Erkennen von solchen Meßergebnissen, die durch den Hook-Effekt falsche Werte vortäuschen, ist ein wesentlicher Fortschritt, da alle Vorteile der Einschritt-Sandwich-Methode (Reaktionszeitverkürzung, Empfindlichkeit, Genauigkeit etc.) ausgenützt werden könnten, ohne das Risiko eines falschen Ergebnisses durch den Hook-Effekt in Kauf nehmen zu müssen.

K.L. Hoffmann et al (Clinical Chemistry, Vol. 30, Nr. 9, 1499, (1984) beschreiben, daß eine Vielzahl von Versuchen unternommen wurden, um das Auftreten des Hook-Effektes zu verhindern. So wird z.B. eine schrittweise Analyse vorgeschlagen, bei der der Analyt zuerst mit einem Rezeptor an der festen Phase reagiert, dann wird die feste Phase extensiv gewaschen, gefolgt von einer zweiten Inkubation mit löslichem markiertem Rezeptor und schließlich wird ein zweitesmal gewaschen, um die gebundenen markierten Rezeptoren von den ungebundenen abzutrennen. Weiter wird eine Verringerung der Probengröße, das Durchführen des Tests mit verschiedenen Verdünnungen des Analyten oder der Gebrauch nur eines kleinen Bereiches der Eichkurve vorgeschlagen. Empfohlen wird auch die Verwendung von Markern mit geringer Aktivität, um auf diese Weise hohe Konzentrationen von markierten Rezeptoren einsetzen zu können, ohne daß dabei die Aktivität (Radio-, Fluoreszenz-, Enzym-, Farbbildungs-Aktivität) zu groß wird.

Zusätzlich beschreiben Hoffmann et al (Clinical Chemistry, Vol. 30, Nr. 9, 1499, (1984) ein Bestimmungsverfahren für einen Einschritt-Sandwich-Test, bei welchem die Zunahme des sich an die feste Phase bindenden Anteils des markierten Rezeptors kinetisch verfolgt wird und diese Werte in einem Computer gespeichert werden. Man berechnet dann die Reaktionsgeschwindigkeiten. Dabei können Meßergebnisse, die durch den Hook-Effekt hervorgerufen wurden, von anderen Werten dadurch unterschieden werden, daß sie wesentlich geringere Zunahmen der Komplexbindung aufweisen. Dieses Verfahren besitzt den Nachteil, daß äußerst aufwendige und teure Computerverfahren verwendet werden müssen, um zu erkennen, ob bei der Bestimmung ein Hook-Effekt vorliegt. Zudem ist dieses Verfahren für enzymimmunologische Bestimmungen ungeeignet.

Es ist daher ein Ziel der Erfindung ein Verfahren zur Verfügung zu stellen, bei welchem durch den Hook-Effekt hervorgerufene falsche Meßergebnisse leicht erkannt werden können, ohne daß dabei die den bekannten Verfahren innewohnenden Nachteile, wie Verlust an Empfindlichkeit, Genauigkeit bzw. erhöhter Aufwand an Arbeit, an Reagenz und an Apparaten in Kauf genommen werden müssen.

Dies wird erfindungsgemäß erreicht durch ein Verfahren zur Bestimmung eines bindefähigen Analyten nach dem Prinzip des heterogenen Immunoassays durch Inkubation einer Probelösung, die den Analyten enthält mit einem mit dem Analyten bindefähigen, spezifischen, in gelöster Phase vorliegenden, markierten ersten Rezeptor und einem in einer festen Phase vorliegenden zweiten Rezeptor, der mit dem ersten Rezeptor nicht kreuzreagiert und einen Komplex, der Analyt und ersten Rezeptor enthält, fixieren kann, Trennung der Phasen nach der Inkubation und quantitative Messung der an die feste Phase gebundenen Markierung, welches dadurch gekennzeichnet ist, daß man die Rückdissoziationsgeschwindigkeit der an die feste Phase gebundenen Markierung in die gelöste Phase bestimmt und den Quotienten von Rückdissoziationsgeschwindigkeit und Meßwert als Maß für die Richtigkeit des Testergebnisses der ersten Messung verwendet.

Im üblichen Sandwich-Test werden nach Abtrennung der Proben- und Reagenzflüssigkeit die Festphasen-fixierten Sandwichkomplexe über die Menge der gebundenen markierten Rezeptoren (radioaktiv, enzymatisch, fluorimetrisch oder ähnlich markiert) bestimmt. Durch diese Abtrennung und Entfernung von nicht gebundenem markierten Rezeptor wird das Gleichgewicht der Analyt-Rezeptor-Bindung gestört und es beginnt eine Rückdissoziation der Sandwich-Komponenten, welche letzlich die Anwesenheit von nicht Festphasen- fixierten markierten Rezeptor-Molekülen in der flüssigen Phase zur Folge hat. Der Rückdissoziationsgrad kann durch Bestimmung der Markierung und Phasentrennung in der festen oder abgetrennten flüssigen Phase ermittelt werden. Als flüssige Phase kommen z.B. die Waschflüssigkeit und bei Enzymimmunoassays die Substratlösung für die Enzymbestimmung in Frage. In dieser Substratlösung wird erfindungsgemäß zunächst der zum Zeitpunkt der Phasentrennung entstandene Meßwert (Farbsignal) bestimmt und anschließend die Kinetik der weiteren Farbbildung als Maß für die Rückdissoziation der Markierung bestimmt.

Da die durch Rückdissoziation des Analyt-Rezeptor-Komplexes bedingte Freisetzung einer Markierungsmenge näherungsweise als Reaktion erster Ordnung abläuft, ist sie der Analytmenge bzw. dem dazugehörigen Meßsignal proportional. Somit kann die freigesetzte Markierungsmenge durch einen Quotienten Alpha = Veränderung des Meßwerts nach Phasentrennung pro Zeiteinheit dividiert durch den Meßwert ausgedrückt werden. Überraschenderweise ist dann, wenn ein Hook-Effekt auftritt, der Quotient Alpha deutlich verändert gegenüber den Fällen, in denen kein Hook-Effekt auftritt.

Somit kann mit Hilfe des Quotienten Alpha entschieden werden, ob ein erhaltener Meßwert mit Hilfe der Eichkurve in eine Analytmenge umgerechnet werden darf oder ob der Meßwert durch einen Hook-Effekt erzeugt worden ist und somit einer Analytmenge oberhalb des obersten Standards zugeschrieben werden muß.

Analyt und Rezeptor können im Rahmen des erfindungsgemäßen Verfahrens prinzipiell alle Substanzpaarungen sein, die miteinander bindefähig sind. In dieser Definition sind im Rahmen der Erfindung außer immunologisch bindefähigen Substanzpaaren auch solche Substanzpaarungen eingeschlossen, die sich analog verhalten. Neben dem in erster Linie in Betracht kommenden und bevorzugt eingesetzten Substanzpaar Antigen-Antikörper (die Bezeichnung Antikörper umfaßt hier auch die bekannten immunologisch aktiven Fragmente sowohl polyklonaler, als auch monoklonaler Herkunft) fallen hierunter auch die Substanzpaarungen Protein A-Immunoglobulin G, Avidin-Biotin, Concanavalin A-Lectin sowie DNA-DNA (Hybrid-Bindung). Alle diese Substanzpaarungen lassen sich nach dem Prinzip des heterogenen Immunoassay (Sandwich-Test) unter einstufiger Inkubation nach dem erfindungsgemäßen Verfahren bestimmen.

Als Rezeptor kommen im Rahmen der Erfindung die Komponenten der obengenannten spezifisch bindefähigen Substanzpaare als solche in Betracht oder Derivate derselben, insbesondere die häufig als Konjugate bezeichneten Derivate der bindefähigen Substanzen, die durch kovalente Bindung an ein Markierungsmolekül gebildet werden. Ein im Rahmen der Erfindung bevorzugtes Beispiel eines derartigen Rezeptors, der als Konjugat vorliegt, ist ein Antikörper oder Antikörper-Fragment, welcher bzw. welches kovalent mit einem Markierungsenzym verbunden ist. Anstelle eines Markierungsenzyms können aber auch Farbstoffmoleküle, Farbbildungskomponenten, insbesondere Fluoreszenzfarbstoffmoleküle oder andere, für ein Nachweissystem geeignete Substanzen verwendet werden. Ein derartiges Konjugat kann auch radioaktiv markiert sein.

Alternativ kann auch der Antikörper sebst radioaktive Atome als Markierung enthalten. In diesem Falle besteht der markierte erste Rezeptor z.B. aus einem radioaktiven Antikörper.

Der zweite, an die feste Phase gebundene Rezeptor kann ebenfalls aus einem Partner der oben angegebenen bindefähigen Substanzpaare als solchem oder einem Derivat davon bestehen. Der Festphasen-gebundene zweite Rezeptor dient beim Sandwich-Assay dazu, den Komplex aus Analyt und erstem Rezeptor an eine feste Phase zu fixieren und somit leicht von der flüssigen Phase abtrennbar zu machen. Hierzu kann der zweite Rezeptor entweder direkt mit dem zu bestimmenden Analyten binden, wobei er in diesem Falle gegen eine andere Determinante des Analyten gerichtet ist wie der erste Rezeptor und infolgedessen vorzugsweise aus einem monoklonalen Antikörper oder einem Fragment davon besteht. Der zweite Rezeptor kann aber auch gegen eine andere, nicht am Analyten selbst vorhandene Determinante des Komplexes aus Analyt und erstem Rezeptor gerichtet sein. Bei dieser Ausführungsform wird vorzugsweise noch ein dritter Rezeptor verwendet, der ebenfalls spezifisch für den Analyten ist und eine andere antigene Determinante erkennt als der erste Rezeptor. Analyt, erster Rezeptor und dritter Rezeptor bilden dann einen Komplex und in diesem Falle wird als zweiter Rezeptor vorzugsweise ein Antikörper verwendet, welcher mit dem dritten Rezeptor bindet. In einer besonders bevorzugten Ausführungsform enthält der erste spezifische Rezeptor ein Antikörperfragment insbesondere ein Fab-Fragment und der dritte Rezeptor besteht aus einem kompletten Antikörper, wobei in diesem Falle der dritte Rezeptor und der Antikörperanteil des ersten Rezeptors monoklonal sind. Der zweite Rezeptor wird in diesem Fall zweckmäßig gegen einen Teil des dritten Rezeptors, besonders bevorzugt gegen den Fc-Teil des dritten Rezeptors gerichtet, wenn der erste Rezeptor ein Fab-Fragment enthält. Es ist auch möglich, den dritten Rezeptor zu derivatisieren und einen zweiten Rezeptor zu verwenden, der gegen diese derivatisierte Stelle gerichtet ist.

Die für das erfindungsgemäße Verfahren wichtige Rückdissoziationsgeschwindigkeit der an die feste Phase gebundenen Markierung wird bestimmt durch die Bindungsfestigkeit zwischen einem der gegen den Analyten gerichteten Rezeptoren und dem Analyten selbst. Gemäß einer bevorzugten Ausführrungsform der Erfindung wird die Rückdissoziationsgeschwindigkeit für zumindest eine Analyt-Konzentration auf einen mittleren Wert eingestellt, d.h. daß weder eine zu hohe noch eine zu geringe Rückdissoziationsgeschwindigkeit erwünscht ist. Ist die Geschwindigkeit zu hoch, so werden die Meßintervalle notwendigerweise immer kürzer, was zu Ungenauigkeiten führt. Eine zu geringe Dissoziationsgeschwindigkeit erhöht die zur Durchführung des Verfahrens erforderliche Zeit erheblich und erschwert das Erkennen einer erniedrigten Rückdissoziationsrate, wie sie bei Vorliegen des Hook-Effekts auftritt. Da das Verfahren vorzugsweise auf üblichen Analysenautomaten durchgeführt wird, läßt sich dann die Kapazität des Automaten nicht mehr voll ausnützen.

Als mittlere Rückdissoziationsgeschwindigkeit wird eine solche verstanden, welche Messungsintervalle zwischen etwa 20 Sekunden und 60 Minuten ermöglicht. Eine Dissoziationsgeschwindigkeit in diesem Bereich läßt sich erreichen durch entsprechende Auswahl der Bindefestigkeit zwischen einem Rezeptor und seinem Bindungspartner, dem Analyt, beispielsweise also durch geeignete Auswahl der Bindungsfestigkeit der Antikörper, oder/und durch die Bindefähigkeit beeinflussende Zusätze im Reaktionsmedium wie oberflächenaktive Substanzen und Salze. Insbesondere werden hierzu Detergenzien oder/und chaotrope Ionen verwendet. Vorzugsweise stimmt man dabei die verschiedenen Komponenten und den vorgesehenen Meßzeitraum so aufeinander ab, daß mindestens bei einer Analytkonzentration innerhalb dieses Zeitraums (Meßintervall) wenigstens 10% der an die feste Phase gebundenen Markierung wieder abdissoziieren.

Bei der bevorzugten Ausführungsform der Erfindung unter Verwendung eines enzymmarkierten ersten Rezeptors wird zweckmäßig nach der Inkubation aller Komponenten und Trennung der Phasen die abgetrennte feste Phase mit einer Lösung eines Farbreagenz zur Bestimmung des Markierungsenzym inkubiert, anschließend wird die Farbreagenzlösung wieder von der festen Phase getrennt und in der abgetrennten Lösung die Rückdissoziationsgeschwindigkeit durch mindestens zwei Messungen in einem zeitlichen Abstand von 20 Sekunden bis 60 Minuten ermittelt. Diese Ausführungsform beruht darauf, daß während des Kontakts zwischen Farbreagenzlösung und fester Phase die Gesamtmenge des an die feste Phase gebundenen Markierungsenzyms und eventuell während dieser Inkubation abdissoziierendes Markierungsenzym zur Farbbildung beitragen. Nach Phasentrennung erfolgt eine weitere Farbbildung, die nur noch auf das abdissoziierte Markierungsenzym zurückzuführen ist. Die abdissoziierte Menge an Markierungsenzym (die der abdissoziierten Menge an Komplex aus Analyt und erstem Rezeptor entspricht) läßt sich daher durch zwei Messungen leicht bestimmen.

Gemäß einer weiteren Ausführungsform der Erfindung wird die feste Phase nach der ersten Phasentrennung anstelle mit einer Substratlösung im Falle des enzymmarkierten Rezeptors erneut mit einer flüssigen Phase inkubiert und nach erneuter Phasentrennung entweder die Abnahme der Markierung an der festen Phase oder die in die flüssige Phase übergegangene Markierung bestimmt. Diese Ausführrungsform eignet sich insbesondere für radioaktive Markierungen oder Farbmarkierungen bzw. Fluoreszenzmarkierung.

Das erfindungsgemäße Verfahren läßt sich prinzipiell auf alle Sandwich-Testsysteme anwenden. Voraussetzung ist lediglich eine meßbare Rückdissoziation des markierten ersten Rezeptors nach Abtrennung des nicht gebundenen, in der flüssigen Phase verbliebenen ersten Rezeptors.

Die Rahmenbedingungen des erfindungsgemäßen Verfahrens werden so gewählt, daß mit und ohne Hook-Effekt gut meßbare Rückdissoziationen auftreten.

Die nachstehenden Beispiele erläutern die Erfindung in Verbindung mit der Zeichnung. In dieser stellen dar:

Fig. 1 eine graphische Darstellung der Abhängigkeit der Extinktion (Meßwert) von der Konzentration des Analyten. Der markierte Teil der Kurve stellt die Eichkurve zur Konzentrationsbestimmung des Analyten dar.

Fig. 2 ein Einsatzelement eines Zentrifugalanalysenautomaten, welches für das erfindungsgemäße Verfahren geeignet ist.

*Beispiel 1*

Bestimmung von AFP

A) Herstellung der Reagenzlösungen

1) Substratpuffer
70 mmol/1 HEPES/NaOH pH 7,0
154 µmol/l Natriumchlorid
3 g/l Rinderserumalbumin,
5 mmol/l Chlorphenolrot-β-D-galactosid (hergestellt nach DE 33 45 748)
2 g/l Tween 20 (nichtionogenes Detergenz)

2) Rezeptor-1-Lösung:
Als Rezeptor 1 wird ein monoklonaler Maus-Anti-AFP-Antikörper eingesetzt, der eine andere antigene Determinante als Rezeptor 3 erkennt. Eine diesen Antikörper enthaltende Ascitesflüssigkeit wird ad 1,8 mol/l mit Ammoniumsulfat versetzt. Das Präzipitat wird in 15 mmol/l Natriumphosphatpuffer, pH 7,0, enthaltend 50 mmol/l Natriumchlorid aufgenommen. Die so erhaltene Lösung wird einer Passage über DEAE-Zellulose unterworfen. Der vollständige Antikörper wird in bekannter Weise zum Fab-Fragment gespalten. Die erhaltenen Fab-Fragmente werden mit β-Galactosidase gekoppelt nach der Methode von E. Ishikawa, J. of Immunoassay 4 (1983), S. 209-327.

3) Rezeptor-2-Lösung:
Schaf-anti-Maus-Fc γ-Antiserum wird wie unter 2) angegeben, aufgereinigt und ebenfalls einer Passage über DEAE-Zellulose unterworfen.

4) Rezeptor-3-Lösung:
Als Rezeptor 3 wird ein monoklonaler Maus-anti-AFP-Antikörper, welcher eine andere Determinante als Rezeptor 1 erkennt, eingesetzt. Eine diesen Antikörper enthaltende Ascitesflüssigkeit wird ad 1,8 mol mit Ammoniumsulfat versetzt. Das Präzipitat wird in 15 mmol/l Natriumphosphatpuffer, pH 7,0, enthaltend 50 mmol/l Natriumchlorid aufgenommen. Die so erhaltene Lösung wird einer Passage über DEAE-Cellulose unterworfen.

B) Herstellung von Reagenzträgern
1) Reagenzträger 1:
40 µl einer Lösungen, die pro Liter
100 mmol Natriumphosphat pH 7,3 (37°C),
2 mmol Magnesiumchlorid,
9 g Natriumchlorid,
5 g Rinderserumalbumin,
5 mg Anti-AFP-monoklonale Antikörper aus Maus (Rezeptor-3-Lösung),
2000 U Anti-AFP Antikörper-(Maus)-Fab-Fragment-β-Galactosidase-Konjugat (Rezeptor-1-Lösung);
Aktivität bestimmt mit ortho-Nitrophenyl-β-D-galactosid bei 37°C
enthält, wird auf ein Vlies aufgetropft, das aus kommerziellem Polyesterpapier besteht. Anschließend wird bei Raumtemperatur getrocknet. Diese Vliese werden bis zu ihrer Verwendung bei 4°C und einer relativen Luftfeuchtigkeit von 20% aufbewahrt.

2) Reagenzträger 2:
Auf ein Zellulose-Vlies werden nach dem Bromcyan-Aktivierungsverfahren (DE-OS 1768512) Schaf-Antikörper gegen den Fcγ-Teil von Maus-Antikörpern (Rezeptor-2-Lösung) fixiert, wobei pro g Fasermaterial 10 µg Antikörper zur Fixierung angeboten werden. Durch Waschen wird ungekoppelter Antikörper entfernt und das Vlies schonend bei Raumtemperatur getrocknet. Die Lagerung der so erhaltenen Vliese folgt analog Reagenzträger 1.

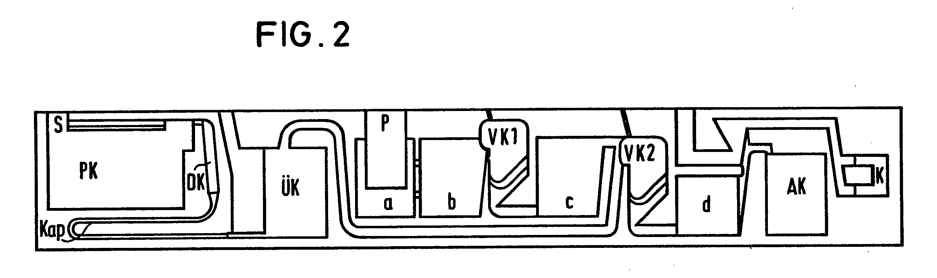

Die Bestimmung mit Hilfe dieser beiden Reagenzträger 1 und 2 erfolgt mit der in der DE-A1 3 425 008.5 beschriebenen Vorrichtung zur Durchführung analytischer Bestimmungen (Fig. 2).
Diese lehrt ein Rotoreinsatzelement für Zentrifugalanalysenautomaten, bestehend aus einem Formkörper, der eine Probenauftragskammer, die mit einer Mehrzahl von Reagenzfeldern in Verbindung steht, die jeweils ein mit einem bestimmten Reagenz imprägniertes saugfähiges Trägermaterial enthalten, wenigstens eine Mischventilkammer und eine Meßkammer aufweist, die zusammen einen Probenflüssigkeitstransportweg bilden, der von radial innen nach radial außen führt, wenn das Einsatzelement auf dem Rotor befestigt ist und weiter wenigstens eine weitere Kammer für die Aufnahme einer Flüssigkeit und einen Transportweg, der von dieser Kammer zur Meßführung führt und mit dem Probenflüssigkeitstransportweg mindestens teilweise identisch ist, aufweist. Dabei führt der Probenflüssigkeitstransportweg von einer Probenauftragskammer (P) über eine mit saugfähigem Material gefüllte, Puffer enthaltende Kammer (a), eine Kammer (c) und eine zwischen den Kammern (a) und (c) angeordnete erste Ventilkammer (Vk1) zu einer zweiten Ventilkammer (Vk2) und von dieser über die Kammer (d) und über eine Auffangkammer (AK) zur Meßkammer (K). Zur Aufnahme einer weiteren Flüssigkeit ist eine als Pumpenkammer ausgebildete Substratkammer (PK) vorgesehen, welche über eine aus Dosierkammer (DK) und Kapillare (Kap) bestehende Dosiereinrichtung und eine Überlaufkammer (ÜK) mit der zweiten Ventilkammer (Vk2) verbunden ist. Fig. 2 zeigt schematisch das verwendete Rotoreinsatzelement.

Reagenzträger 1 wird auf Feld c des Rotoreinsatzelements plaziert und Reagenzträger 2 auf Feld d. Dabei werden 40 µl Probe durch eine Öffnung am oberen Rand direkt auf das Feld a pipettiert. Die Probe ist 1:5 mit 0,9% NaCl-Lösung verdünnt 270 µl Substratlösung werden in Kammer PK pipettiert. Durch ein geeignetes Zentrifugations-Programm, bei dem hohe Drehzahlen mit Stillstand abwechseln, werden dann Probe und Substratlösung in Richtung Trennmatrix und Küvette gefördert.

Dabei werden im Verlauf des Programms die Rezeptoren 1 und 3 durch die Probenflüssigkeit vom Feld c eluiert und die homogene Mischung anschließend zur Reaktion gebracht. Auf Feld d werden die gebildeten Komplexe an den Rezeptor 2 gebunden. Der Transfer der Probe von Feld c nach d erfolgt innerhalb sehr kurzer Zeit.

Die Substratlösung wird durch die Dosierkammer DK in Portionen geteilt, von denen die ersten zum Auswaschen von überschüssigem, nicht komplexiertem Konjugat dienen.

Die über Komplexbildung auf d gebundene β-Galactosidase-Aktivität ist proportional zur in der Probe enthaltenen AFP-Menge. Diese Aktivität wird mit einer weiteren Substratportion bestimmt, wobei das Substrat in einer 5minütigen Reaktion zu farbigen Produkten umgesetzt wird. Die gebildete Farbe und die weitere Farbentwicklung/min in der flüssigen Phase werden in der Küvette bei 600 nm gemessen.

Unter diesen Bedingungen wurden folgende Resultate erhalten:

### A F P

| IU/ml | 0 | 60 | 135 | 200 | 50.000 | 100.000 | 200.000 | 500.000 | 1.000.000 |
|---|---|---|---|---|---|---|---|---|---|
| Meßwert (mE) | 274 | 1516 | 2783 | 5363 | $\geq 8.000$ | 6587 | 4670 | 2782 | 2069 |
| Steigung $\left(\dfrac{mE}{min}\right)$ | 19 | 131 | 217 | 305 | — | 48 | 40 | 30 | 32 |
| $\alpha =$ Steigung/ Meßwert $\times$ 100 | 6,9 | 8,6 | 7,8 | 5,7 | — | 0,7 | 0,9 | 1,1 | 1,5 |

Hook-Effekt!

Alle Messungen wurden bei 600 nm bei einer Schichtdicke von 0,3 cm durchgeführt und auf Schichtdicke d = 1 cm umgerechnet.

*Beispiel 2*

(IgE-Bestimmung):

Es wurde analog Beispiel 1 gearbeitet mit folgenden Unterschieden:
a) Als Rezeptor 3 wird ein gegen human IgE gerichteter monoklonaler Maus-anti-IgE-Antikörper verwendet.

b) Als Rezeptor 1 wird ein weiterer gegen IgE gerichteter monoklonaler Maus-anti-IgE-Antikörper, der jedoch eine andere Antigendeterminante als Rezeptor 3 erkennt, verwendet.
c) Bei der Herstellung des Reagenzträgers 1 werden 12 mU/Test an Rezeptor-1-Lösung eingesetzt.
d) Die Probe wird 1:3 mit NaCl-Lösung verdünnt.

Unter den entsprechenden Bedingungen wurden folgende Ergebnisse erhalten:

| U/ml | 0 | 52 | 90 | 182 | 278 | 383 | 489[1] | 12.200[1] | 29.750 | 64.500 | 118.000 |
|---|---|---|---|---|---|---|---|---|---|---|---|
| Meßwert (mE)[2] | 88 | 604 | 1098 | 2050 | 2898 | 3646 | 4073 | 6445 | 4573 | 3151 | 2230 |
| Steigung[2] (mE/min) | 4 | 34 | 60 | 101 | 127 | 146 | 150 | 68 | 65 | 98 | 38 |
| $\alpha = \dfrac{\text{Steigung}}{\text{Meßwert}} \times 100$ | 4,5 | 5,6 | 5,5 | 4,9 | 4,4 | 4,0 | 3,7 | 1,1 | 1,4 | 1,5 | 1,7 |

Hook-Effekt!

1) Bei Konzentrationen zwischen etwa 500 und 12.000 U/ml war der Photometer-Meßbereich überschritten.
2) Alle Messungen erfolgten bei 600 nm mit einer 0,3 cm-Küvette, die Extinktionen wurden anschließen auf 1 cm Lichtweg umgerechnet.

Die letzten drei Spalten zeigen Werte, die durch den Hook-Effekt hervorgerufen wurden und für eine quantitative Bestimmung des Analyten nicht verwendet werden können.

*Beispiel 3*

Mikrotiterplatten (MTPs) werden pro Vertiefung mit je 200 µl einer Lösung eines monoklonalen Maus-anti-AFP-Antikörpers Rezeptor-2-Lösung in der Konzentration 1 µg Protein/ml in Puffer A (20mM Carbonatpuffer, pH 9,6) für 1 h bei Raumtemperatur (RT) beschichtet. Anschließend werden nichtspezifische Bindungsstellen durch Nachinkubation der MTPs mit 300 µl Puffer B (50 mM Kaliumphosphatpuffer, pH 7,5, 154 mM NaCl, 1% RSA, 1 g/l Tween 20) für 1/2 h bei RT abgesättigt.

Nach Waschen mit 300 µl Puffer C (100 mM HEPES/NaOH, pH 7,25, 154 mM NaCl, 2,5 g/l Schafnormal-IgG, 0,5 mM Mg-L-Aspartat, 2 g/l Tween 20) werden pro Vertiefung 50 µl Probe und 200 µl einer Enzym-Antikörper-Konjugat-Lösung (Rezeptor-1-Lösung; β-Galaktosidase-Konjugat eines Fab-Fragmentes von einem weiteren monoklonalen Maus-anti-AFP-Antikörper, welcher den Antikörper von Rezeptor 2 nicht behindert; 0,5 U/ml; Messung der Aktivität mit o-Nitrophenyl-β-D-Galaktosid bei 37°C) in Puffer C zugegeben und für 1 h bei RT inkubiert. Anschließend werden die Flüssigkeiten aus den MTP-Vertiefungen verworfen, die Vertiefungen mit 300 μl Puffer B gewaschen und danach mit 250 μl Substratlösung (5 mM Chlorphenolrot-β-D-Galaktosid, 70 mM HEPES/NaOH, pH 7,0, 154 mM NaCl, 3 g/l RSA, 2 g/l Tween 20) für 2,5 h bei RT inkubiert.

Nach Ablauf dieser Inkubationszeit werden aus jeder Vertiefung der MTP je 100 μl entnommen und in die Näpfe einer weiteren MTP überführt. Sofort im Anschluß hieran findet eine Absorptionsmessung (Messung $t_1$) mittels eines handelsüblichen MTP-Meßgerätes statt (= 570 nm, bichromatische Korrektur bei 630 nm), nach einer weiteren Stunde Inkubationszeit bei RT erfolgt eine zweite Absorptionsmessung (Messung $t_2$) unter den gleichen Bedingungen wie Messung $t_1$. Die Absorptionsdifferenz zwischen Messung $t_2$ Messung $t_1$ ist proportional der mit der Substratlösung transferierten Enzymmenge. Die Ergebnisse eines typischen Versuchs sind in folgender Tabelle dargestellt:

| (AFP) in IU/ml | 1,5 | 10 | 40 | 156 | 625 | 2.500 | 10.000 | 40.000 |
|---|---|---|---|---|---|---|---|---|
| $mE_{t1}$ | 67 | 419 | 1720 | >[2] | >[2] | >[2] | 1643 | 485 |
| $mE_{t2} - mE_{t1}$ | 32 | 171 | 540 | — | — | — | 141 | 51 |
| $\alpha$[1] | 478 | 408 | 314 | — | — | — | 86 | 105 |

Die letzten zwei Spalten zeigen das Vorliegen eines Hook-Effektes an.

1) $\alpha = \dfrac{mE_{t2} - mE_{t1}}{mE_{t1}} \times 100$

2) = oberhalb des Photometermeßbereichs (200 mE)

## Patentansprüche

1. Verfahren zur Bestimmung eines bindefähigen Analyten nach dem Prinzip des heterogenen Immunoassay durch Inkubation einer Probelösung, die den Analyten enthält mit einem mit dem Analyten bindefähigen, spezifischen, in gelöster Phase vorliegenden, markierten ersten Rezeptor und einem in einer festen Phase vorliegenden zweiten Rezeptor, der mit dem ersten Rezeptor nicht kreuzreagiert und einen Komplex, der Analyt und ersten Rezeptor enthält, fixieren kann, Trennung der Phasen nach der Inkubation und quantitative Messung der an die feste Phase gebundenen Markierung, dadurch gekennzeichnet, daß man die Rückdissoziationsgeschwindigkeit der an die feste Phase gebundenen Markierung in die gelöste Phase bestimmt und den Quotienten von Rückdissoziationsgeschwindigkeit und Meßwert als Maß für die Richtigkeit des Testergebnisses der ersten Messung verwendet.

2. Verfahren nach Anspruch 1, dadurch gekennzeichnet, daß man die Rückdissoziationsgeschwindigkeit durch mindestens eine zweite Messung der an die feste Phase gebundenen Markierung in einem zeitlichen Abstand von 20 Sekunden bis 60 Minuten zur ersten Messung ermittelt.

3. Verfahren nach Anspruch 1 oder 2, dadurch gekennzeichnet, daß man einen enzymmarkierten ersten Rezeptor verwendet, die feste Phase mit einer Lösung eines Farbreagenz zur Bestimmung des Markierungsenzyms inkubiert, dann die Reagenzlösung von der festen Phase trennt und in der Reagenzlösung durch mindestens zwei Messungen in einem zeitlichen Abstand von 20 Sekunden bis 60 Minuten die Rückdissoziationsgeschwindigkeit ermittelt.

4. Verfahren nach Anspruch 1 oder 2, dadurch gekennzeichnet, daß man nach der ersten Messung die feste Phase erneut mit einer flüssigen Phase inkubiert und die Rückdissoziationsgeschwindigkeit durch Messung der an der festen Phase verbliebenen Markierung oder der in die flüssige Phase übergegangenen Markierung bestimmt.

5. Verfahren nach einem der vorhergehenden Ansprüche, dadurch gekennzeichnet, daß man einen dritten, für den Analyten spezifischen Rezeptor zusetzt, welcher eine andere antigene Determinante des Analyten erkennt, als der erste Rezeptor und daß der zweite Rezeptor mit dem dritten Rezeptor bindefähig ist.

6. Verfahren nach Anspruch 5, dadurch gekennzeichnet, daß man als dritten Rezeptor einen monoklonalen Antikörper verwendet, der erste Rezeptor ein Fab-Fragment eines monoklonalen Antikörpers enthält und der zweite Rezeptor mit dem Fc-Teil des dritten Rezeptors bindefähig ist.

7. Verfahren nach einem der vorhergehenden Ansprüche, dadurch gekennzeichnet, daß die Rückdissoziationsgeschwindigkeit mindestens eines Rezeptors im Reagenz durch Auswahl des Antikörpers nach seiner Bindungskonstante oder/und Zugabe von Detergenzien oder/und chaotroper Ionen eingestellt wird.

8. Verfahren nach Anspruch 7, dadurch gekennzeichnet, daß man die Rückdissoziationsgeschwindigkeit mindestens eines Rezeptors so auswählt, daß wenigstens bei einer Konzentration des Analyten im Meßzeitraum mindestens 10% der an die feste Phase gebundenen Markierung wieder dissoziieren.

## Claims

1. Process for the determination of a bindable analyte according to the principle of heterogeneous immunoassay by incubation of a sample solution which contains the analyte with a labelled first receptor specifically bindable with the analyte and present in dissolved phase and a second receptor present in a solid phase which does not cross-react with the first receptor and can fix a complex which contains the analyte and first receptor, separation of the phases after incubation and quantitative measurement of the labelling bound to the solid phase, characterised in that one determines the back dissociation velocity of the labelling bound to the solid phase into the dissolved phase and uses the quotients of back dissociation velocity and measurement value as measure for the correctness of the test result of the first measurement.

2. Process according to claim 1, characterised in that one determines the back dissociation velocity by at least one second measurement of the labelling bound to the solid phase a the time interval of 20 seconds to 60 minutes after the the first measurement.

3. Process according to claim 1 or 2, characterised in that one uses an enzyme-labelled first receptor, incubates the solid phase with a solution of a colour reagent for the determination of the labelling enzyme, then separates the reagent solution from the solid phase and determines the back dissociation velocity in the reagent solution by at least two measurements at a time interval of 20 seconds to 60 minutes.

4. Process according to claim 1 or 2, characterised in that, after the first measurement, one again incubates the solid phase with a liquid phase and determines the back dissociation velocity by measurement of the labelling remaining on the solid phase or of the labelling which has passed over into the liquid phase.

5. Process according to one of the preceding claims, characterised in that one adds a third receptor specific for the analyte which recognises a determinant of the analyte different from the first receptor and that the second receptor is bindable with the third receptor.

6. Process according to claim 5, characterised in that, as the third receptor, one uses a monoclonal antibody, the first receptor contains a Fab fragment of a monoclonal antibody and the second receptor is bindable with the Fc part of the third receptor.

7. Process according to one of the preceding claims, characterised in that the back dissociation constant of at least one receptor in the reagent is adjusted by choice of the antibody according to its binding constant and/or the addition of detergents and/or chaotropic ions.

8. Process according to claim 7, characterised in that the back dissociation constant of at least one receptor is so chosen that, at least at one concentration of the analyte, in the measurement time at least 10% of the labelling bound to the solid phase again dissociates.

## Revendications

1. Procédé pour la détermination d'un «analyte» apte à se lier selon le principe de l'immunoessai hétérogène par incubation d'une solution d'échantillon qui contient l'analyte avec un premier récepteur marqué, apte à se lier avec l'analyte, spécifique, se présentant en phase dissoute et avec un deuxième récepteur se présentant dans une phase solide, qui ne réagit pas de façon croisée avec le premier récepteur et peut fixer un complexe qui contient l'analyte et le premier récepteur, séparation des phases après l'incubation et mesure quantitative du marquage lié à la phase solide, caractérisé en ce qu'on détermine la vitesse de redissociation du marquage lié à la phase solide dans la phase dissoute et en ce que l'on utilise le quotient de la vitesse de redissociation et de la valeur mesurée comme mesure pour l'exactitude du résultat d'essai de la première mesure.

2. Procédé selon la revendication 1, caractérisé en ce qu'on détermine la vitesse de redissociation par au moins une deuxième mesure du marquage lié à la phase solide dans un intervalle de temps de 20 secondes à 60 minutes par rapport à la première mesure.

3. Procédé selon la revendication 1 ou 2, caractérisé en ce qu'on utilise un premier récepteur marqué par une enzyme, incube la phase solide avec une solution d'un réactif coloré pour la détermination de l'enzyme de marquage, puis sépare la solution de réactif de la phase solide et détermine la vitesse de redissociation dans la solution de réactif par au moins deux mesures dans un intervalle de temps de 20 secondes à 60 minutes.

4. Procédé selon la revendication 1 ou 2, caractérisé en ce qu'on incube, après la première mesure, la phase solide de nouveau avec une phase liquide et en ce qu'on détermine la vitesse de redissociation par mesure du marquage restant sur la phase solide ou du marquage transféré dans la phase liquide.

5. Procédé selon l'une des revendications précédentes, caractérisé en ce qu'on ajoute un troisième récepteur, spécifique de l'anayte, lequel récepteur reconnaît un déterminant antigénique autre que le premier récepteur et en ce que le deuxième récepteur est apte à se lier avec le troisième récepteur.

6. Procédé selon la revendication 5, caractérisé en ce qu'on l'utilise, comme troisième récepteur, un anticorps monoclonal, en ce que le premier récepteur contient un fragment Fab d'un anticorps monoclonal et en ce que le deuxième récepteur est apte à se lier avec la partie Fc du troisième récepteur.

7. Procédé selon l'une des revendications précédentes, caractérisé en ce que l'on règle la vitesse de redissociation d'au moins un récepteur dans le réactif en choisissant l'anticorps selon sa constante de liaison ou/et par addition de détergents ou/et d'ions chaotropes.

8. Procédé selon la revendication 7, caractérisé en ce qu'on choisit la vitesse de redissociation d'au moins un récepteur, de sorte qu'au moins pour une concentration de l'analyte d'au moins 10% pendant la durée de l'analyte le marquage lié à la phase solide se dissocie de nouveau.

# FIG.1

# FIG.2